# EUROPÄISCHE PATENTSCHRIFT

(11) **EP 3 656 189 B1**
(45) Veröffentlichungstag und Bekanntmachung des Hinweises auf die Patenterteilung: **14.01.2026**
(21) Anmeldenummer: 18743675.3
(22) Anmeldetag: 05.07.2018
(51) Int. Cl.: H05H 1/24, A61N 1/44

(54) **PLASMA-BEHANDLUNGSGERÄT**
PLASMA TREATMENT DEVICE
DISPOSITIF DE TRAITEMENT AU PLASMA

(30) Priorität: 19.07.2017 DE 102017116305
(43) Veröffentlichungstag der Anmeldung: 27.05.2020
(73) Patentinhaber: Cinogy GmbH, 37115 Duderstadt (DE)
(72) Erfinder: WANDKE, Dirk, 37308 Heilbad Heiligenstadt (DE); HAHNL, Mirko, 37339 Berlingerode (DE); STORCK, Karl-Otto, 37115 Duderstadt (DE); TRUTWIG, Leonhard, 37115 Duderstadt (DE); RICKE, Melanie, 37191 Katlenburg-Lindau (DE)
(74) Vertreter: Gramm, Lins & Partner Patent- und Rechtsanwälte PartGmbB
(86) Internationale Anmeldenummer: PCT/DE2018/100619
(87) Internationale Veröffentlichungsnummer: WO 2019/015717

(56) Entgegenhaltungen:
- WO-A2-2016/186501
- DE-A1- 102012 025 079
- DE-A1- 102014 220 488
- DE-A1- 4 040 911
- JP-A- H0 863 539
- US-A1- 2012 232 540
- US-A1- 2016 287 310
- US-A1- 2017 094 769

## Beschreibung

Die Erfindung betrifft ein Plasma-Behandlungsgerät zur Durchführung einer dielektrisch behinderten Plasmabehandlung einer Oberfläche.

Durch DE 10 2014 013 716 A1 ist ein derartiges Plasma-Behandlungsgerät bekannt. Die Elektrodeneinheit ist dabei als flächige Einheit mit einer flächigen Elektrodenanordnung und einem flächigen Dielektrikum ausgebildet. Die Materialien können dabei so gewählt sein, dass die Elektrodeneinheit, die auf eine Wundfläche oder Hautfläche auflegbar ist, sich flexibel der Oberfläche anpassen kann. Die Elektrodeneinheit ist dabei mit einem Ansatz versehen, in den sich sowohl die Elektrodenanordnung als auch das Dielektrikum erstreckt. Der Ansatz der Elektrodeneinheit ist in eine Aufnahme einer Versorgungseinheit einführbar und kann dort mit einem Hebel-Klemmmechanismus mechanisch gehalten werden. Dabei wird zugleich die Elektrodenanordnung mit der Versorgungseinheit elektrisch kontaktiert. Die Versorgungseinheit enthält dabei eine Hochspannungsstufe, die aus einer zugeführten Netzspannung die für die dielektrisch behinderte Plasmabehandlung benötigte Hochspannung, regelmäßig in Form von Hochspannungs-Wechselimpulszügen, generiert.

Bei dem bekannten Behandlungsgerät ist die Elektrodeneinheit auswechselbar mit der Versorgungseinheit verbindbar, weil die Elektrodeneinheit zum Einmalgebrauch vorgesehen ist. Dies hat den Vorteil, dass die Elektrodeneinheiten steril produziert und verpackt werden können und Sterilisierungsmaßnahmen vor oder nach dem Gebrauch der Elektrodeneinheit nicht erforderlich sind.

Die DE 10 2014 220 488 A1 offenbart eine Vorrichtung zum Erzeugen eines kalten Atmosphärenplasmas. Die Vorrichtung weist hierfür wenigstens eine erste Elektrodenschicht und eine zweite Elektrodenschicht auf, wobei zwischen der ersten Elektrodenschicht und der zweiten Elektrodenschicht eine Dielektrikumsschicht angeordnet ist. Die Elektrodenschichten weisen dabei jeweils wenigstens eine Elektrode auf, sodass durch Anlegen einer elektrischen Spannung an die Elektroden ein schwaches Atmosphärenplasmas erzeugt werden kann. Jede Vorrichtung kann dabei einen Informationsträger zum Speichern von Betriebsparametern aufweisen.

Der vorliegenden Erfindung liegt die Aufgabe zugrunde, ein Plasma-Behandlungsgerät der vorbekannten Art so auszugestalten, dass es für ein breiteres Anwendungsgebiet verwendbar und einfach handhabbar ist.

Die Erfindung löst das Problem durch ein Plasma-Behandlungsgerät mit den Merkmalen von Anspruch 1.

Der vorliegenden Erfindung liegt der Gedanke zugrunde, dass die durch die lösbare Verbindung zwischen Versorgungseinheit und Elektrodeneinheit bewirkte Austauschbarkeit der Elektrodeneinheit grundsätzlich eine Verwendung derselben Versorgungseinheit mit unterschiedlichen Elektrodeneinheiten ermöglicht. Insbesondere können bei einer Wundversorgung unterschiedlich große Elektrodeneinheiten verwendet werden, um unterschiedlich große Wundflächen mit dem dielektrisch behinderten Plasma zu behandeln und dadurch die Keime in der Wundfläche zu reduzieren und die Heilung durch eine Anregung der Mikrozirkulation im Wundbereich zu fördern. Wenn allerdings unterschiedlich große Elektrodeneinheiten mit der Versorgungseinheit verbunden werden, entsteht das Problem, dass die Versorgungseinheit Spannungsimpulse mit konstanten Energieinhalten produziert, obwohl unterschiedlich große Elektrodeneinheiten unterschiedlich große Energiezufuhren benötigen. Durch die erfindungsgemäße Codierung der Elektrodeneinheit ist es nunmehr möglich, unterschiedlich große Elektrodeneinheiten an die Versorgungseinheit anzuschließen und die Elektrodeneinheit mit der an ihre Größe angepassten Energiemenge zu versorgen.

In gleicher Weise ist es möglich, aufgrund der Codierung der Elektrodeneinheit die von der Versorgungseinheit zugeführte Spannung an die Elektrodeneinheit anzupassen, wenn eine Elektrodeneinheit für eine Wundversorgung, eine andere Elektrodeneinheit hingegen für eine kosmetische Behandlung der Hautoberfläche vorgesehen und ausgebildet ist. Auf diese Weise können für die jeweilige Elektrodeneinheit geeignete Behandlungsprogramme durch die Versorgungseinheit zur Verfügung gestellt werden. Es ist daher möglich, die erfindungsgemäße Codierung nicht nur für unterschiedliche Elektrodengrößen vorzunehmen, sondern auch für unterschiedlich ausgebildete Elektroden, die für spezielle Anwendungsfälle angepasst sind, beispielsweise durch unterschiedliche Ausführungen der Behandlungsseite der Elektrodeneinheit.

Ferner ist es möglich, die der Elektrodeneinheit zugeführte Hochspannung daran anzupassen, ob auf der Behandlungsseite hautpflegende oder heilende Substanzen angeordnet werden, wie dies beispielsweise durch DE 10 2015 111 401 B3 oder durch DE 10 2013 019 057 A1 bekannt ist. In gleicher Weise kann ferner der möglichen Zuführung von flüssigen oder gasförmigen Substanzen durch isolierte Kanäle im Dielektrikum (vgl. DE 10 2014 013 716 A1) Rechnung getragen werden.

Die erfindungsgemäße Codierung ist an der Elektrodeneinheit ausgebildet.

Weist die Elektrodeneinheit einen Ansatz auf, der in eine, beispielsweise schlitzförmige Aufnahme der Versorgungseinheit einführbar ist und die Codierung trägt, können mechanische Verbindung, elektrische Kontaktierung und die Übertragung der Codierung durch die Ausbildung des Ansatzes erfolgen. In einer mechanischen Ausbildung der Codierung können diese die Form von nebeneinander angeordneten Erhöhungen haben und die Erkennungseinrichtung mit durch die Erhöhung betätigbaren Kippschaltern ausgebildet sein. In dieser Ausführungsform können die Erkennung der Codierung und die entsprechende Steuerung der Versorgungsspannung durch die Kippschalter erfolgen, wenn die Kippschalter zum Umschalten der Versorgungsspannung in der Versorgungseinheit ausgelegt sind.

Alternativ können die Erhöhungen auch ohne Stromversorgung ausgewertet werden, indem sie beispielsweise in der Versorgungseinheit Piezoelemente beaufschlagen und so die Codierung in Spannungssignale umwandeln.

Ist die Codierung in optischer Form ausgebildet und durch eine optische Erkennungseinrichtung in der Versorgungseinheit erkennbar, wird für die Erkennungseinrichtung eine Spannungsversorgung benötigt.

Dies ist ebenfalls der Fall, wenn die Elektrodeneinheit für die Codierung einen Transponder enthält, der mittels der Erkennungseinrichtung der Versorgungseinheit drahtlos abfragbar ist. Um sicherzustellen, dass die Erkennungseinrichtung auch tatsächlich nur die Elektrodeneinheit erkennt, mit der die Versorgungseinheit mechanisch und elektrisch verbunden ist, kann in der Versorgungseinheit ein Detektor vorgesehen sein, der bei einer hergestellten Verbindung zwischen der Versorgungseinheit und der Elektrodeneinheit ein Abfragesignal für die Erkennungseinrichtung generiert. Dadurch ist sichergestellt, dass die Abfrage erst stattfindet, wenn die Elektrodeneinheit mit der Versorgungseinheit verbunden ist.

Eine weitere Möglichkeit zur Erkennung der Codierung und Steuerung der Versorgungsspannung besteht darin, dass die Codierung mittels wenigstens eines Permanentmagneten in der Elektrodeneinheit erfolgt, mit dem wenigstens einen Schalter der Versorgungseinheit betätigbar ist, wenn die Elektrodeneinheit mit der Versorgungseinheit verbunden wird.

Es ist ohne weiteres ersichtlich, dass die Verbindung der Elektrodeneinheit mit der Versorgungseinheit in jeder dem Fachmann geläufigen Weise erfolgen kann, wenn dabei eine berührungssichere elektrische und eine sich nicht ungewollt lösende mechanische Verbindung hergestellt wird. Die Erfindung ist daher nicht auf eine spezielle konstruktive Ausbildung der Elektrodeneinheit und/oder der Versorgungseinheit beschränkt.

Die Versorgungseinheit kann an eine übliche Stromversorgung mittels eines Kabels angeschlossen sein. Möglich ist ferner, dass der Versorgungseinheit bereits die Hochspannung zugeführt wird, die in der Versorgungseinheit dann entsprechend der Codierung lediglich modifiziert wird. Von Bedeutung ist ferner, dass die Versorgungseinheit ferner autark ausgebildet sein kann, indem sie die benötigten Hochspannungssignale aus einer Batteriespannung generiert. Die Batterien sind dabei zweckmäßigerweise in der Versorgungseinheit selbst angeordnet.

Die Erfindung soll im Folgenden anhand eines nicht beschränkenden Ausführungsbeispiels näher erläutert werden. Es zeigen:
- Figur 1a): einen Horizontalschnitt durch ein Ausführungsbeispiel einer Versorgungseinheit und eine Draufsicht auf eine Elektrodeneinheit;
- Figur 1b): einen Vertikalschnitt entlang der Linie A-A aus Figur 1a) durch die Versorgungseinheit und eine Seitenansicht der Elektrodeneinheit;
- Figur 1c): eine Draufsicht auf die Versorgungseinheit und die Elektrodeneinheit, jeweils im noch nicht verbundenen Zustand;
- Figur 2a) bis 2c): Darstellungen gemäß den Figuren 1a) bis 1c), jedoch im verbundenen Zustand von Versorgungseinheit und Elektrodeneinheit;
- Figur 3a) bis 3c): die Darstellungen gemäß Figur 1a) bis 1c) nur für die Versorgungseinheit im Ruhezustand;
- Figur 4a) und 4b): das Ausführungsbeispiel gemäß Figur 1 mit einer schematischen Darstellung der Elektronik im nicht verbundenen Zustand von Versorgungseinheit und Elektrodeneinheit;
- Figur 5a) und 5b): die den Figur 4 entsprechenden Darstellungen im verbundenen Zustand von Elektrodeneinheit und Versorgungseinheit;
- Figur 6: ein Querschnitt zur Verdeutlichung der Kippschalterstellungen im verbundenen Zustand von Elektrodeneinheit und Versorgungseinheit;
- Figur 7a) und 7b): Schnittdarstellungen eines zweiten Ausführungsbeispiels mit einer magnetischen Betätigung von Mikroschaltern im nicht verbundenen Zustand von Elektrodeneinheit und Versorgungseinheit;
- Figur 8a) und 8b): die Darstellungen gemäß Figur 7 im verbundenen Zustand von Elektrodeneinheit und Versorgungseinheit;
- Figur 9: eine Querschnittsdarstellung gemäß Figur 6 für das zweite Ausführungsbeispiel;
- Figur 10a) und 10b): eine Darstellung eines dritten Ausführungsbeispiels mit einer optischen Erkennung im nicht verbundenen Zustand von Versorgungseinheit und Elektrodeneinheit;
- Figur 11: die Darstellungen gemäß Figur 10 im verbundenen Zustand von Versorgungseinheit und Elektrodeneinheit.

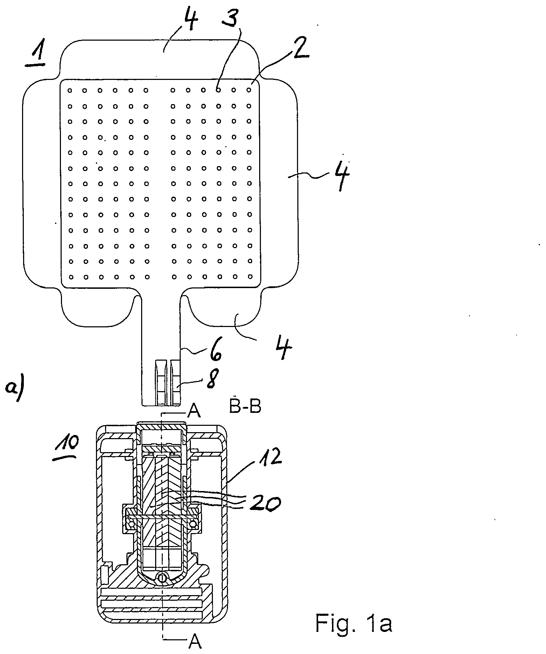

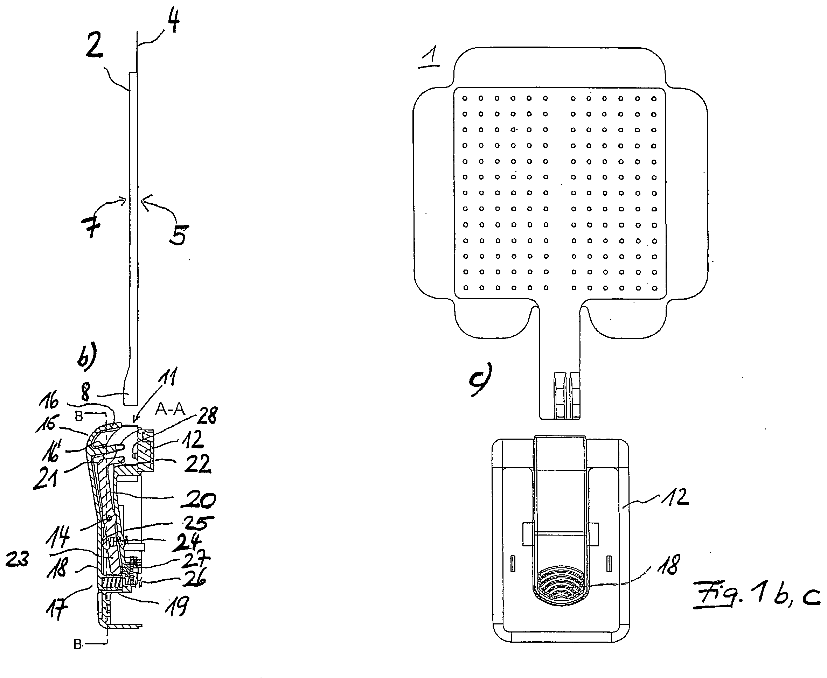

In den Figuren 1 bis 3 sind in erster Linie die mechanisch konstruktiven Merkmale einer ersten Ausführungsform dargestellt. Figur 1 zeigt eine Elektrodeneinheit 1, die flächig ausgebildet ist. In der Zeichnung ist lediglich ein Dielektrikum 2 dargestellt, in dem sich über die Fläche verteilt Durchgangsöffnungen 3 befinden, durch die bei der Verwendung der Elektrodeneinheit 1 als Wundauflage Wundsekret abgesaugt oder in anderen Behandlungsfällen ein Gas oder eine Flüssigkeit auf die Hautoberfläche geleitet werden kann. Das Dielektrikum 2 weist dünne flexible Ansätze 4 auf, die auf einer Behandlungsseite 5 der Elektrodeneinheit 1 klebend ausgebildet sind, um so nach Art eines Pflaster die Elektrodeneinheit 1 auf der Haut eines menschlichen Körpers zu befestigen. Da von der Elektrodeneinheit 1 nur Ansichten dargestellt sind und das Dielektrikum 2 eine Elektrodenanordnung allseitig umgibt, ist die Elektrodenanordnung in den Figuren 1 bis 3 nicht dargestellt.

Die Elektrodeneinheit 1, also das Dielektrikum 2 mit der darin eingebetteten Elektrodenanordnung, ist flächig ausgebildet. Demgemäß weist die Elektrodeneinheit eine große Behandlungsseite und eine große gegenüberliegende Oberseite 7 auf, deren Abmessungen groß sind gegenüber der Höhe, also dem Abstand zwischen Behandlungsseite 5 und Oberseite 7. Vorzugsweise ist das Material des Dielektrikums 2 und der darin eingebetteten Elektrode flexibel, sodass sich die Elektrodeneinheit 1 an eine unregelmäßige Hautoberfläche anpassen kann.

In den Ansatz 6 erstreckt sich sowohl das Dielektrikum 2 als auch die darin eingebettete Elektrodenanordnung. Auf der Oberseite sind am freien Ende des Ansatzes 6 zwei stegförmige Erhöhungen 8 nebeneinander dargestellt, die etwa zwei Drittel der Breite des Ansatzes 6 in Anspruch nehmen. Über die Breite des Ansatzes 6 könnten somit drei derartige Erhöhungen 8 angeordnet sein. Das Vorhandensein einer Erhöhung 1 entspricht einer digitalen "1", das Fehlen der Erhöhung einer digitalen "0". Mit drei Bits lassen sich bekanntlich 2³ = 8 verschiedene Codierungen realisieren. In vielen Fällen wird diese Anzahl der Codierungsmöglichkeiten nicht benötigt, sodass im Einzelfall auch nur zwei Erhöhungen (vier verschiedene Codierungen) oder nur eine Erhöhung (zwei verschiedene Codierungen) verwendet werden können. Selbstverständlich kann die Anzahl der Erhöhungen 8 auch erhöht werden, wenn dies erforderlich erscheint.

Das Dielektrikum 2 ist vorzugsweise durch einen gießfähigen oder spritzgussfähigen Kunststoff gebildet. Die eingebettete Elektrode kann eine flexible Metallfolie, aber auch eine dünne Schicht eines mit leitfähigen Zusätzen versehenen Kunststoffs sein. Vorzugsweise sind das Material des Dielektrikums 2 und der eingebetteten Elektrode von gleicher Art, beispielsweise beide Silikone.

**In** dem dargestellten Ausführungsbeispiel sind die Erhöhungen 8 in Form einer Rampe ausgebildet, deren Funktion nachstehend noch näher erläutert wird.

Die Elektrodeneinheit 1 ist mit einer Versorgungseinheit 10 verbindbar. Die Verbindung erfolgt über den Ansatz 6, für dessen Aufnahme die Versorgungseinheit 10 eine schlitzförmige Aufnahme 11 in einem Gehäuse 12 aufweist. Die schlitzförmige Aufnahme 11 ist mittels eines zweiarmigen Betätigungshebels 13 verschließbar oder freigebbar. Der zweiarmige Betätigungshebel 13 ist an einer im Gehäuse 12 gelagerten Drehachse 14 drehbar gelagert. Ein vorderes Ende 15 des Betätigungshebels ist zum Verschließen der schlitzförmigen Aufnahme 11 abgekröpft ausgebildet und bildet zwei in Einschubrichtung des Ansatzes 6 hintereinander angeordnete Verschlussstege 16, 16'. Am anderen Arm des zweiarmigen Hebels 13 befindet sich am hinteren Ende 17 eine gerillte Tastenfläche 18 mit der der Betätigungshebel 13 mit seinem hinteren Ende 17 in das Gehäuse gegen die Kraft einer Rückstellfeder 19 gedrückt wird. Diese gedrückte Stellung ist in Figur 1b) dargestellt. In dieser Stellung ist die schlitzförmige Aufnahme 11 geöffnet und ermöglicht das Einschieben des Ansatzes 6 der Elektrodeneinheit 1.

Abgedeckt von dem Betätigungshebel, der sich im Wesentlichen über die Breite der Drehachse 14 erstreckt, sind an derselben Drehachse 14 drei Kipphebel 20 drehbar gelagert, deren Breite der Breite der Erhöhungen 8 entspricht. Auch die Kipphebel 20 sind als zweiarmige Hebel ausgebildet und weisen an ihrem vorderen Ende 21 eine Abkröpfung 22 auf, die auf der rampenförmigen Erhöhung 8 als Tasthebel gleiten kann.

Der ein hinteres Ende 23 jenseits der Drehachse 14 bildende andere Arm des zweiarmigen Kipphebels 20 stützt sich an dem Gehäuse 12 mittels einer Feder 24 ab, durch die das vordere Ende 21 in Richtung des Verschlusses der schlitzförmigen Aufnahme 11 vorgespannt ist. Über einen Wulst 25 liegt das hintere Ende 23 des Kipphebels 20 an der Unterseite des Betätigungshebels 13 an, sodass die Kipphebel beim Drücken auf die Tastenfläche 18 mit dem Betätigungshebel 13 verschwenkt werden.

Das hintere Ende 23 des Kipphebels 20 wirkt ferner auf einen ihm zugeordneten Schalter 26 auf einer Platine 27 innerhalb des Gehäuses 12 ein.

Die schlitzförmige Aufnahme 11 bildet einen Einschubkanal, auf dessen Boden sich ein Kontaktvorsprung, der mit einem entsprechenden Gegenkontakt an der Unterseite des Ansatzes 6 zusammenwirkt. Der Ansatz 6 weist auf seiner Unterseite entsprechende Gegenkontakte auf, die im eingeschobenen Zustand der elektrischen Kontaktierung der Elektrodeneinheit 1 mit der Versorgungseinheit 10 dienen. Der Kontaktvorsprung 28 ist - in Figur 1 nicht dargestellt - mit einem Elektronikteil der Versorgungseinheit 10 verbunden.

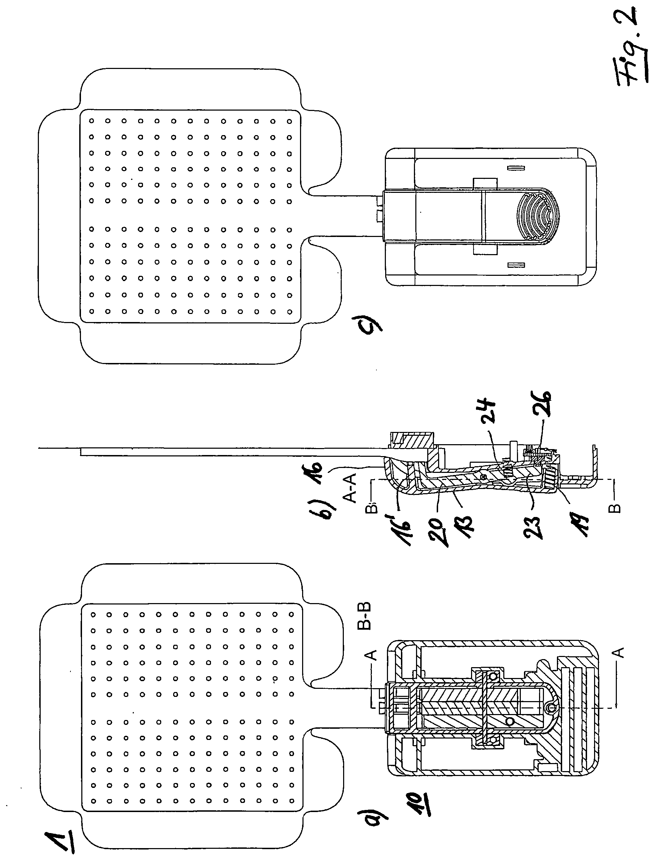

Figur 2 verdeutlicht den eingeschobenen Zustand der Elektrodeneinheit 1 in die Versorgungseinheit 10. Insbesondere Figur 2a) lässt erkennen, dass die Rückstellfeder 19 den Betätigungshebel 13 gegen den Ansatz 6 drückt, wobei der vordere Verschlusssteg 16 die rampenförmige Erhöhung 8 hintergreift. Zusätzlich drückt der weitere Verschlusssteg 16' auf die Oberseite der Erhöhung 8 und sichert so die Verriegelung.

Figur 2b) verdeutlicht ferner, dass dort, wo Erhöhungen 8 vorhanden sind, die zugehörigen Kipphebel 20 am vorderen Ende gegen die Rückstellkraft der Feder 24 gedrückt werden, sodass das hintere Ende 23 des Kipphebels 20 den zugehörigen Schalter 26 betätigt. Die in dieser Ausführungsform vorhandenen drei Schalter 26 setzten somit das Vorhandensein der als Codierung wirkenden Erhöhungen 8 in entsprechende elektrische Schaltsignale um.

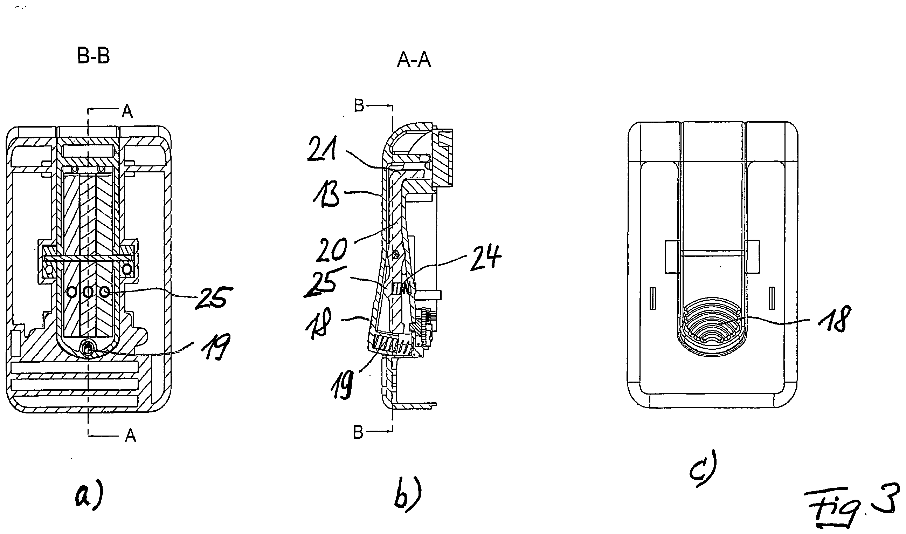

Die Figuren 3a) bis 3c) verdeutlichen die Ruhestellung der Versorgungseinheit 10 in der die Rückstellfeder 19 den Betätigungshebel 13 in eine die schlitzförmige Aufnahme 11 vollständig verschließende Stellung drückt und das vordere Ende der Kipphebel 20 ebenfalls in Richtung des Bodens der schlitzförmigen Aufnahme 11 durch die Feder 24 gedrückt wird. Dabei ist erkennbar, dass durch Druck auf die Tastenfläche 18 des Betätigungshebels 13 nicht nur der Betätigungshebel 13 gedreht wird, sondern über den Wulst 25 auch die drei Kipphebel 20.

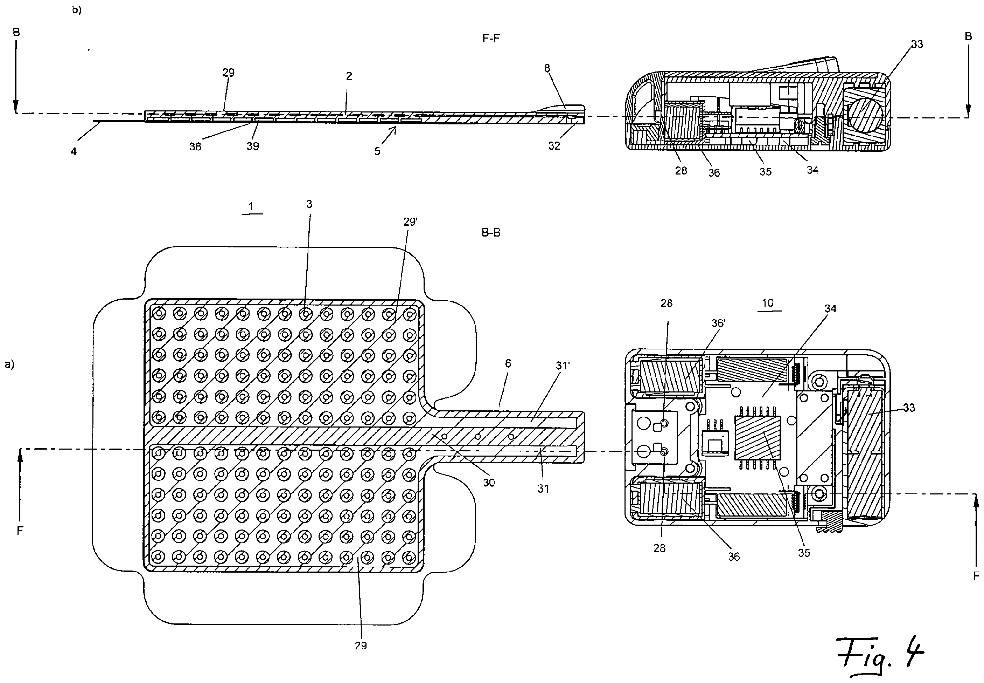

Die Figuren 4 bis 6 zeigen das erste Ausführungsbeispiel mit einer schematischen Darstellung der Elektronik. Der Vertikalschnitt in Figur 4a) gemäß der Schnittlinie F-F in Figur 4b) und der Horizontalschnitt B-B entsprechend der Schnittlinie in Figur 4a) lassen erkennen, dass in der Elektrodeneinheit eine Elektrodenanordnung aus zwei flächigen Elektroden 29, 29' gebildet ist, wobei die Elektroden 29, 29' spiegelsymmetrisch zueinander ausgebildet sind. Die beiden Elektroden sind durch einen durch das Dielektrikum 2 gebildeten mittleren Isoliersteg 30 voneinander getrennt, der sich in den Ansatz 6 hinein erstreckt. In gleicher Weise erstrecken sich die Elektroden 29, 29' in den Ansatz 6 und bilden dort Kontaktstreifen 31, 31' beiderseits des Isolierstegs 30.

Figur 4b) lässt erkennen, dass am Ende des Ansatzes 6 das Material des Dielektrikums 2 auf der Unterseite des Ansatzes 6 im Bereich der Kontaktstreifen 31, 31' fehlt und nutförmige Ausnehmungen 32 bildet, in denen die Kontaktstreifen 31, 31' frei zugänglich liegen. Die nutförmigen Ausnehmungen 32 sind so ausgebildet, dass die Kontaktvorsprünge 28 in sie hineinragen, wenn die Elektrodeneinheit 1 in die Versorgungseinheit 10 eingeschoben wird. Am Ende der Einschubbewegung kontaktiert der Kontaktvorsprung 28 den zugehörigen Kontaktstreifen 31, 31', wodurch die elektrische Verbindung zwischen Versorgungseinheit 10 und Elektrodeneinheit 1 hergestellt wird.

Der elektrische Teil der Versorgungseinheit 4 enthält Batterien 33, sodass die Versorgungseinheit 10 gemäß diesem Ausführungsbeispiel autark arbeitet, also keine Zuleitung für eine Versorgungsspannung benötigt. An die Batterien 33 schließt sich eine Platine 34 für die Erstellung einer Zwischenspannung an. In einer durch einen IC-Schaltkreis dargestellten Steuerung 35 wird die Batteriegleichspannung zerhackt und in Spannungsimpulse umgeformt. Diese Spannungsimpulse werden so auf zwei Spulen 36, 36' geleitet, das in diesen Hochspannungsimpulse entstehen. Aufgrund von Schwingvorgängen können die Hochspannungsimpulse mehrere Schwingungen mit abnehmender Amplitude enthalten. Die von den Spulen 36, 36' gebildeten Hochspannungsimpulse sind gegenphasig, sodass die Summe ihrer momentanen Amplituden immer null ergibt. "Null" ist dabei ein Bezugspotential, beispielsweise Masse.

Die gegenphasigen Hochspannungsimpulse gelangen auf die beiden Kontaktvorsprünge 28, sodass die beiden Elektroden 29, 29' jeweils mit Hochspannungsimpulsen versorgt werden, die zueinander gegenphasig und gleich groß sind. Mit diesen Impulsen werden die entsprechenden Plasmafelder unterhalb der Elektroden 29, 29' gebildet.

In dem Horizontalschnitt gemäß Figur 4a) ist noch erkennbar, dass die Elektroden 29, 29' um die Durchgangsöffnungen 3 des Dielektrikums 2 herum ebenfalls Ausnehmungen 37 aufweisen, die jedoch größer sind als die Durchgangsöffnungen 3, sodass im Bereich der Durchgangsöffnungen 3 in den Ausnehmungen 37 Material des Dielektrikums 2 die Durchgangsöffnungen 3 begrenzt. Auf diese Weise wird ein unmittelbarer Kontakt eines Fluids mit den Elektroden 29, 29' vermieden. Ferner ist erkennbar, dass das Dielektrikum 2 auf der Behandlungsseite 5 in dem flächigen Bereich der Elektroden 29, 29' durch Stege 38 begrenzte Kammern 39 ausbildet, die zur Behandlungsseite hin offen sind, horizontal durch die Stege 38 und oberseitig durch das Dielektrikum 2 begrenzt werden. Die Stege 38 können sich kreuzende Stege gleicher Höhe sein, sodass rechteckige oder quadratische Kammern 39 gebildet werden. Die Kammern 39 stellen eine Strukturierung der Behandlungsseite 5 des Dielektrikums 2 dar, durch die sichergestellt wird, dass auf der Behandlungsseite 5 durch die Elektrode in Lufträumen ein Plasma für die Behandlung ausgebildet werden kann. Die dargestellte Strukturierung mittels der Kammern 39 ist lediglich beispielhaft zu verstehen, da auch andere Strukturierungen geeignet sein können, wie beispielsweise Noppen, die zu der zu behandelnden Oberfläche gerichtet sind und mit ihrer Oberseite auf der zu behandelnden Oberfläche anliegen und zwischen sich Lufträume für das Plasma ausbilden.

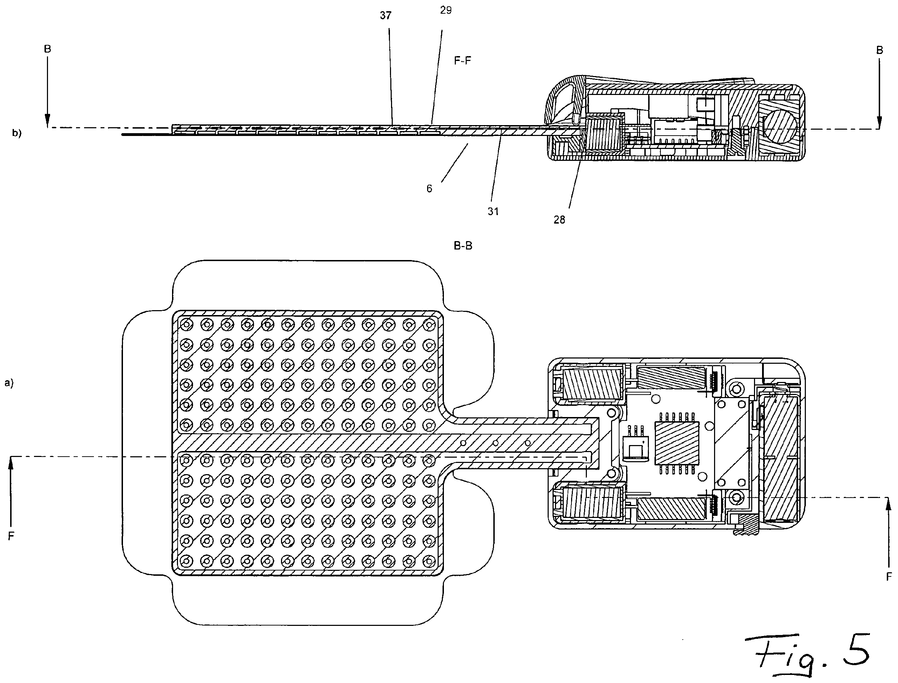

Figur 5a) und 5b) zeigen Elektrodeneinheit 1 und Versorgungseinheit 10 im miteinander verbundenen Zustand. In mechanischer Hinsicht ist dieser Zustand anhand der Figur 2 bereits erläutert worden. Figur 5b) zeigt, wie der (nur halb geschnitten dargestellte) Kontaktvorsprung 28 in die Ausnehmung 32 (vgl. Figur 4b)) auf der Unterseite des Ansatzes 6 eingreift und somit direkt dem Kontaktstreifen 31 der Elektrode 29 kontaktiert.

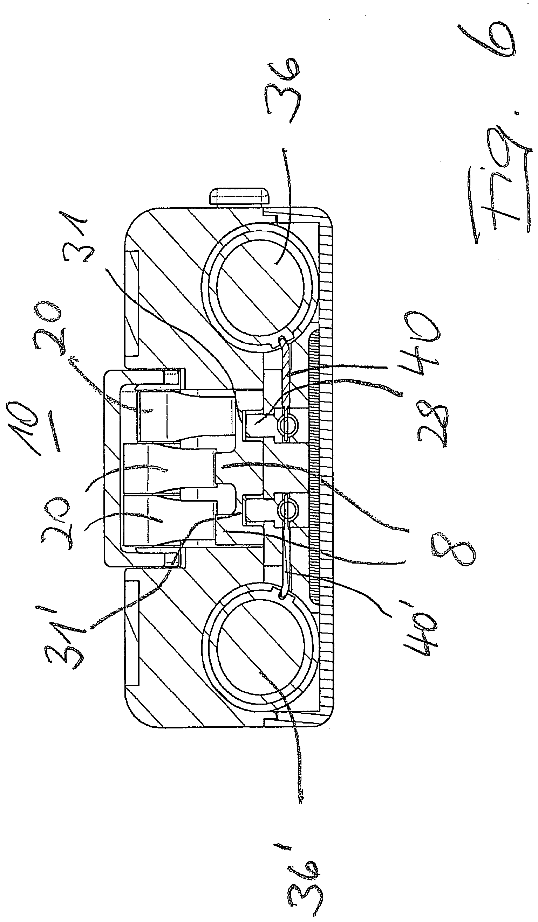

Der Hochschnitt durch die Versorgungseinheit 10 im Bereich der Spulen 36, 36' gemäß Figur 6 verdeutlicht die Codierung durch die Erhöhungen 8 und die von ihnen betätigten oder nicht betätigten Kipphebel 20. Ferner lässt Figur 6 eine für Hochspannungen geeignete Verbindungsleitung 40, 40' zwischen der zugehörigen Spule 36, 36' und dem zugehörigen Kontaktvorsprung 28 erkennen. Der Kontaktvorsprung 28 kontaktiert den Kontaktstreifen 31, 31' der zugehörigen Elektroden 29, 29', sodass die in den Spulen 36, 36' gebildeten Hochspannungsimpulse auf die Elektroden 29, 29' gegenphasig übertragen werden.

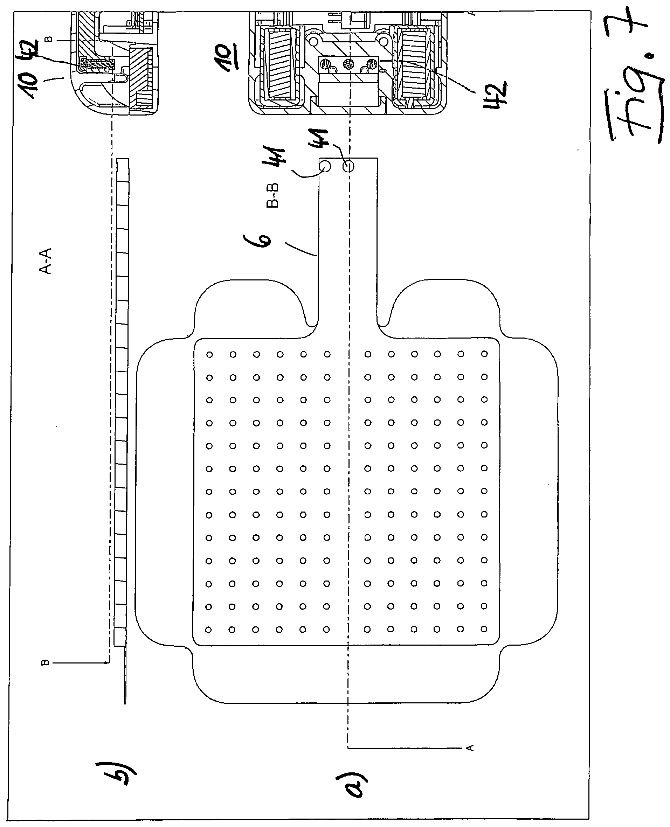

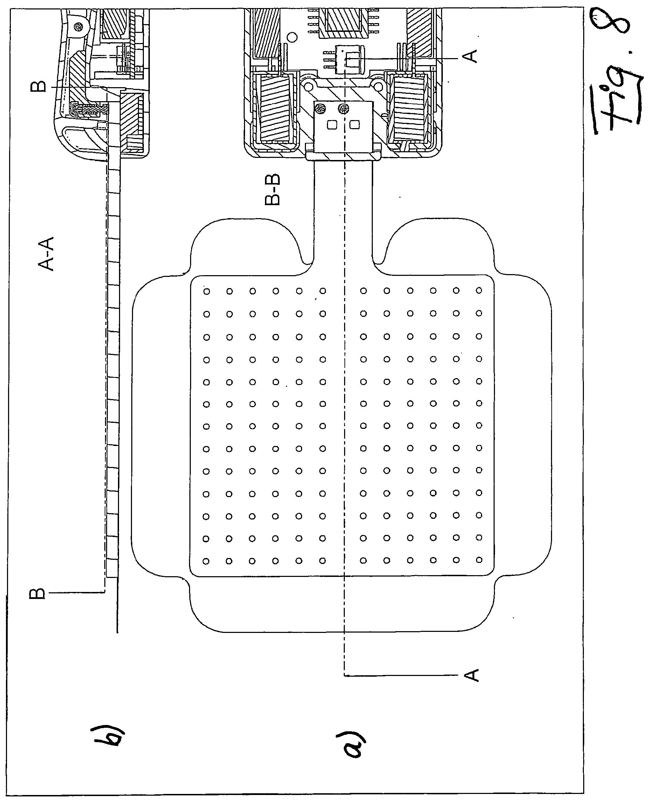

Das in den Figuren 7 und 8 dargestellte zweite Ausführungsbeispiel entspricht konstruktiv im Wesentlichen dem ersten Ausführungsbeispiel und unterscheidet sich von diesem lediglich dadurch, dass eine magnetische Codierung am Ende des Ansatzes 6 vorgesehen ist. Die Erhöhungen 8 des ersten Ausführungsbeispiels sind ersetzt durch die Anordnung zweier kleiner Permanentmagnete 41, denen in der nur im vorderen Teil dargestellten Versorgungseinheit 10 Mikroschalter 42 zugeordnet sind. In dem dargestellten Ausführungsbeispiel sind drei Mikroschalter 42 vorgesehen, die von einem, zwei oder drei Permanentmagneten 41 betätigt werden können. Die Anzahl und Position der Permanentmagnete 41 bewirkt somit die Codierung.

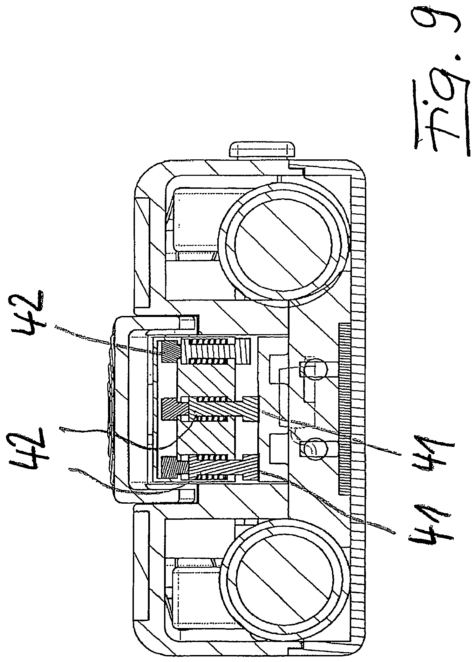

Figur 8 zeigt den verbundenen Zustand von Elektrodeneinheit 1 und Versorgungseinheit 10. Die Hochschnittdarstellung der Figur 9 verdeutlicht die der Codierung gemäß Figur 6 entsprechenden Stellung der Mikroschalter 42, von denen zwei durch die Permanentmagneten 41 in eine Schaltstellung angezogen sind, während einer der Mikroschalter 42 in einer federbelasteten Ruhestellung verharrt, weil für ihn kein Permanentmagnet 41 im Ansatz 6 angeordnet ist.

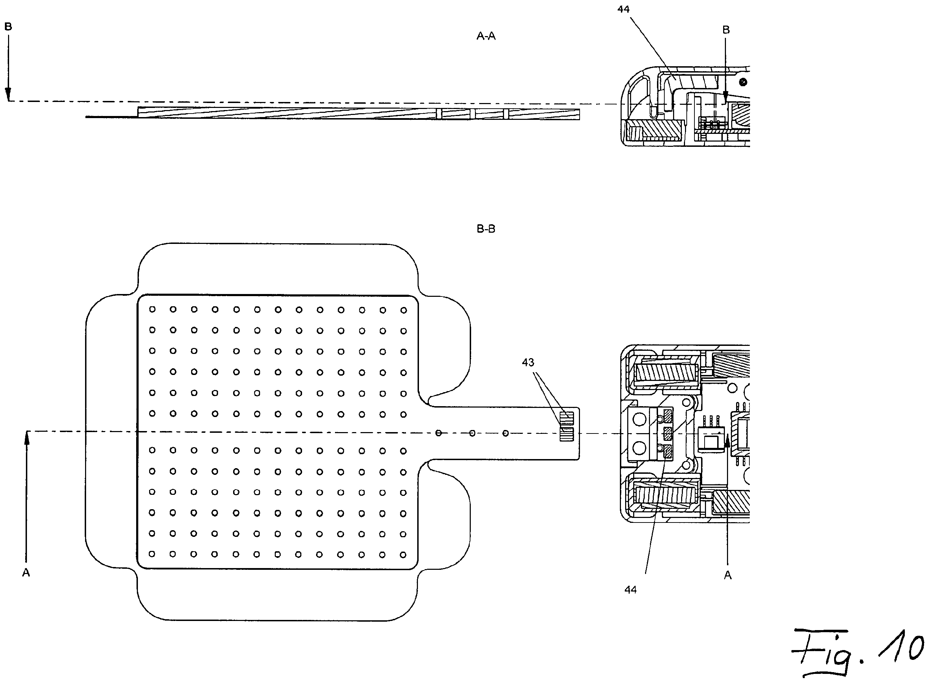

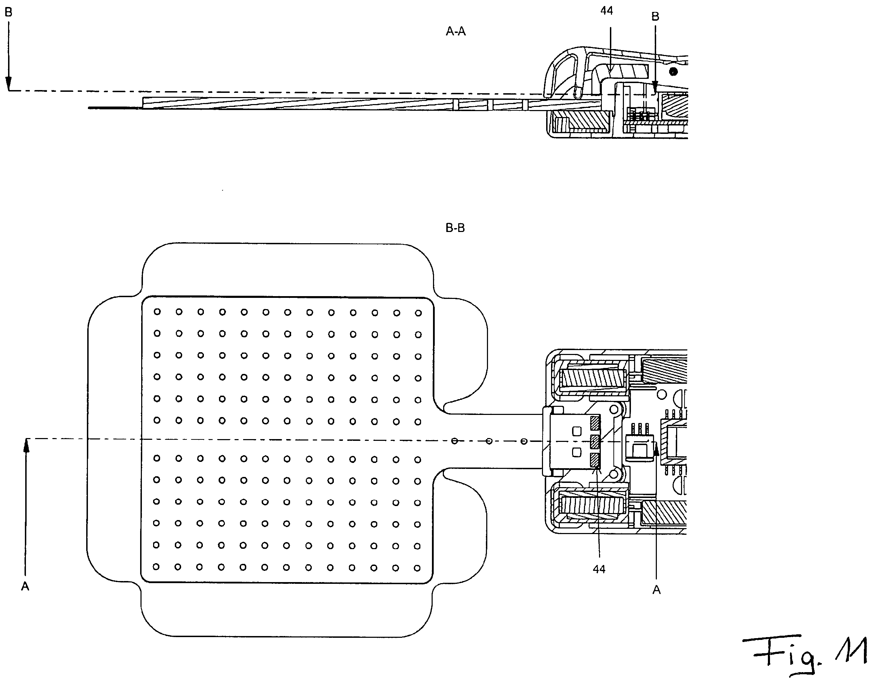

In den in Figur 10 und 11 in gleicher Weise dargestellten dritten Ausführungsbeispiel befindet sich am vorderen Ende des Ansatzes 6 eine optische Codierung 43 und in der Versorgungseinheit eine entsprechende optische Leseeinrichtung 44 mit entsprechenden Abtastpositionen, die den Positionen der optischen Codierung 43 entspricht, wenn die Elektrodeneinheit gemäß Figur 11 in die Versorgungseinheit 10 eingeschoben ist. Die optische Leseeinrichtung 44 erkennt beispielsweise das Vorhandensein einer geschwärzten Fläche als "1"-Signal und eine nicht geschwärzte Fläche als "0"-Signal. Statt dieser optischen Codierung kann in einer Variante der Ansatz 6 auch mit einem Barcode versehen sein und die Versorgungseinheit 10 einen Barcode-Leser aufweisen. Der Barcode selbst kann dann die Information über die Größe der Behandlungsfläche der Elektrodeneinrichtung 1 enthalten.

Es ist ohne weiteres ersichtlich, dass jegliche weitere optische oder andere Codierungen im Rahmen der vorliegenden Erfindung verwirklicht werden können.

## Patentansprüche

1. Plasma-Behandlungsgerät zur Durchführung einer dielektrisch behinderten Plasmaentladung mit
(a) einer eine Behandlungsseite (5) aufweisenden Elektrodeneinheit (1) und
(b) einer Versorgungseinheit (10), mit der die Elektrodeneinheit (1) mechanisch verbindbar und elektrisch kontaktierbar ist, um mit einer für die Plasmaerzeugung benötigte Versorgungsspannung versorgt zu werden,
(c) wobei die Elektrodeneinheit (1) eine Elektrodenanordnung aufweist, die wenigstens zur Behandlungsseite (5) mit einem flächigen Dielektrikum (2) abgeschirmt ist,
(d) wobei durch eine lösbare Verbindung zwischen Versorgungseinheit (10) und Elektrodeneinheit (1) eine Austauschbarkeit der Elektrodeneinheit (1) bewirkt ist, die eine Verwendung derselben Versorgungseinheit (10) mit unterschiedlichen Elektrodeneinheiten (1) ermöglicht, sodass bei einer Wundversorgung unterschiedlich große Elektrodeneinheiten (1) verwendbar werden,
wobei
(e) die Elektrodeneinheiten (1) eine Codierung für ihre Elektrodengröße haben,
(f) die Versorgungseinheit (10) eine Erkennungseinrichtung für die Codierung der Elektrodengröße aufweist und
(g) die Erkennungseinrichtung mit einer Steuereinrichtung verbunden ist, die in Abhängigkeit von der erkannten Codierung der Elektrodengröße die Versorgungsspannung für die Plasmaerzeugung so steuert, dass die Elektrodeneinheit (1) mit der an ihre Größe angepassten Energiemenge versorgt wird.

2. Plasma-Behandlungsgerät nach Anspruch 1, **dadurch gekennzeichnet, dass** die Elektrodeneinheit (1) einen Ansatz (6) aufweist, der in eine Aufnahme (11) der Versorgungseinheit (10) einführbar ist und dass der Ansatz (6) die Codierung trägt.

3. Plasma-Behandlungsgerät nach Anspruch 1 oder 2, **dadurch gekennzeichnet, dass** die Codierung mechanisch in Form von Erhöhungen (8) und die Erkennungseinrichtung mit durch die Erhöhungen (8) betätigbaren Kippschaltern (20) ausgebildet ist.

4. Plasma-Behandlungsgerät nach Anspruch 3, **dadurch gekennzeichnet, dass** die Kippschalter (20) zum Umschalten der Versorgungsspannung in der Versorgungseinheit (10) ausgelegt sind.

5. Plasma-Behandlungsgerät nach Anspruch 1 oder 2, **dadurch gekennzeichnet, dass** die Codierung in optischer Form ausgebildet und die Versorgungseinheit (10) mit einer optischen Erkennungseinrichtung versehen ist.

6. Plasma-Behandlungsgerät nach Anspruch 1 oder 2, **dadurch gekennzeichnet, dass** die Codierung mittels wenigstens eines Permanentmagneten erfolgt, mit dem wenigstens ein Schalter der Versorgungseinheit (10) betätigbar ist.

7. Plasma-Behandlungsgerät nach Anspruch 1 oder 2, **dadurch gekennzeichnet, dass** die Elektrodeneinheit (1) einen Transponder enthält, der mittels der Erkennungseinrichtung der Versorgungseinheit (10) abfragbar ist.

8. Plasma-Behandlungsgerät nach Anspruch 7, **dadurch gekennzeichnet, dass** ein Detektor der Versorgungseinheit (10) die hergestellte Verbindung zwischen Versorgungseinheit (10) und Elektrodeneinheit (1) erkennt und daraufhin ein Abfragesignal für die Erkennungseinrichtung generiert.

## Claims

1. A plasma treatment device for conducting a dielectric barrier plasma discharge with
(a) an electrode unit (1) comprising a treatment side (5) and
(b) a power pack (10) that can be mechanically connected and electrically contacted to the electrode unit (1) in order to supply it with the supply voltage required for plasma generation,
(c) wherein the electrode unit (1) comprises an electrode arrangement that is shielded at least on the treatment side (5) by a flat dielectric (2),
(d) wherein an interchangeability of the electrode unit (1) is effected by a detachable connection between power pack (10) and electrode unit (1), said interchangeability allowing the same power pack (10) to be used with different electrode units (1) such that electrode units (1) of different sizes can be used to treat a wound,
wherein
(e) the electrode units (1) have a coding for their electrode size,
(f) the power pack (10) comprises a detection device for the coding of the electrode size and
(g) the detection device is connected to a control device, which controls the supply voltage for plasma generation as a function of the detected coding of the electrode size in such a way that the electrode unit (1) is supplied with an amount of energy adjusted to its size.

2. The plasma treatment device according to claim 1, **characterised in that** the electrode unit (1) comprises an attachment (6) which can be inserted into a mount (11) of the power pack (10) and that the attachment (6) carries the coding.

3. The plasma treatment device according to claim 1 or 2, **characterised in that** the coding is configured mechanically in the form of elevations (8) and the detection device has toggle switches (20) that can be activated by way of the elevations (8).

4. The plasma treatment device according to claim 3, **characterised in that** the toggle switches (20) are configured to switch the supply voltage in the power pack (10).

5. The plasma treatment device according to claim 1 or 2, **characterised in that** the coding is configured in an optical form and the power pack (10) is fitted with an optical detection device.

6. The plasma treatment device according to claim 1 or 2, **characterised in that** the coding is achieved by means of at least one permanent magnet, with which at least one switch of the power pack (10) can be activated.

7. The plasma treatment device according to claim 1 or 2, **characterised in that** the electrode unit (1) contains a transponder that can be queried by means of the detection device of the power pack (10).

8. The plasma treatment device according to claim 7, **characterised in that** a detector of the power pack (10) detects the connection established between power pack (10) and electrode unit (1) and then generates a query signal for the detection device.

## Revendications

1. Appareil de traitement au plasma pour réaliser une décharge plasma à barrière diélectrique, comprenant
(a) une unité d'électrode (1) présentant un côté traitement (5) et
(b) une unité d'alimentation (10) à laquelle l'unité d'électrode (1) peut être reliée mécaniquement et être connectée électriquement afin d'être alimentée en une tension d'alimentation nécessaire à la génération du plasma,
(c) l'unité d'électrode (1) comportant un ensemble d'électrode qui est protégé au moins du côté traitement (5) par un diélectrique plat (2),
(d) une connexion amovible entre l'unité d'alimentation (10) et l'unité d'électrode (1) permettant l'interchangeabilité de l'unité d'électrode (1), ce qui permet d'utiliser la même unité d'alimentation (10) avec différentes unités d'électrode (1), de sorte que des unités d'électrode (1) de tailles différentes peuvent être utilisées pour le traitement des plaies,
dans lequel
(e) les unités d'électrode (1) ont un codage pour la taille de leur électrode,
(f) l'unité d'alimentation (10) comporte un dispositif de détection du codage de la taille de l'électrode, et
(g) le dispositif de détection est connecté à un dispositif de commande qui, en fonction du codage détecté de la taille de l'électrode, commande la tension d'alimentation pour la génération de plasma de telle sorte que l'unité d'électrode (1) soit alimentée en la quantité d'énergie adaptée à sa taille.

2. Appareil de traitement au plasma selon la revendication 1, **caractérisé en ce que** l'unité d'électrode (1) présente un embout (6) qui peut être introduit dans un logement (11) de l'unité d'alimentation (10), et **en ce que** l'embout (6) porte le codage.

3. Appareil de traitement au plasma selon la revendication 1 ou 2, **caractérisé en ce que** le codage est réalisé mécaniquement sous la forme de reliefs (8), et le dispositif de détection est constitué d'interrupteurs à bascule (20) actionnables par les reliefs (8).

4. Appareil de traitement au plasma selon la revendication 3, **caractérisé en ce que** les interrupteurs à bascule (20) sont conçus pour commuter la tension d'alimentation dans l'unité d'alimentation (10).

5. Appareil de traitement au plasma selon la revendication 1 ou 2, **caractérisé en ce que** le codage est réalisé sous forme optique, et l'unité d'alimentation (10) est équipée d'un dispositif de détection optique.

6. Appareil de traitement au plasma selon la revendication 1 ou 2, **caractérisé en ce que** le codage s'effectue au moyen d'au moins un aimant permanent permettant d'actionneur au moins un interrupteur de l'unité d'alimentation (10).

7. Appareil de traitement au plasma selon la revendication 1 ou 2, **caractérisé en ce que** l'unité d'électrode (1) comprend un transpondeur qui peut être interrogé au moyen du dispositif de détection de l'unité d'alimentation (10).

8. Appareil de traitement au plasma selon la revendication 7, **caractérisé en ce qu'**un détecteur de l'unité d'alimentation (10) détecte la connexion établie entre l'unité d'alimentation (10) et l'unité d'électrode (1) et génère ensuite un signal d'interrogation pour le dispositif de détection.
